(19) **Europäisches Patentamt European Patent Office Office européen des brevets**

(11) **EP 2 124 902 B1**

(12) **EUROPEAN PATENT SPECIFICATION**

(45) Date of publication and mention of the grant of the patent:
**26.08.2015 Bulletin 2015/35**

(21) Application number: **08719155.7**

(22) Date of filing: **22.02.2008**

(51) Int Cl.:
***A61K 9/20*** (2006.01)

(86) International application number:
**PCT/IB2008/000396**

(87) International publication number:
**WO 2008/102248 (28.08.2008 Gazette 2008/35)**

(54) **AN IMPROVED MONOLITHIC DRUG DELIVERY SYSTEM**

VERBESSERTES MONOLITHISCHES ARZNEIAUSGABESYSTEM

DISPOSITIF DE DÉLIVRANCE DE MÉDICAMENTS MONOLITHIQUE AMÉLIORÉ

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.02.2007 ZA 200609747**

(43) Date of publication of application:
**02.12.2009 Bulletin 2009/49**

(73) Proprietor: **University Of The Witwatersrand, Johannesburg Braamfontein Johannesburg (ZA)**

(72) Inventors:
• **SIBAMBO, Sibongile, Ruth**
 **Komatipoort 1340 (ZA)**
• **PILLAY, Vinees**
 **Sandton 2196 (ZA)**
• **CHOONARA, Yahya, Essop**
 **Lenasia Ext 1 1820 (ZA)**

(74) Representative: **Regimbeau**
 **20, rue de Chazelles**
 **75847 Paris Cedex 17 (FR)**

(56) References cited:
**EP-A- 1 356 809      WO-A-95/29664**
**US-A1- 2002 168 410      US-A1- 2003 031 716**

• **WEBBER, W. L. ET AL: "Characterization of soluble , salt loaded, degradable PLGA films and their release of tetracycline" JOURNAL OF BIOMEDICAL MATERIALS RESEARCH PART A, vol. 41, no. 1, 6 December 1998 (1998-12-06), pages 18-29, XP002488224**
• **PRATT, L. ET AL: "The effect of ionic electrolytes on hydrolytic degradation of biodegradable polymers: Mechanical and thermodynamic properties and molecular modeling" JOURNAL OF POLYMER SCIENCE PART A: POLYMER CHEMISTRY, vol. 31, no. 7, 10 March 2003 (2003-03-10), pages 1759-1769, XP002488225**
• **LISA C. DU TOIT ET AL: 'Approaches to Fabricating Anti-TB Nanosystems Embodying a Salted-Out and Cross-linked Architecture' 4TH INTERNATIONAL CONFERENCE ON PHARMACEUTICAL AND PHARMACOLOGICAL SCIENCES (ICPPS) 22 September 2006, XP0055051411**
• **LISA C. DU TOIT ET AL: 'Preliminary production of enteronanoparticles based on a salted-out and cross-linked architecture' 2006 AAPS ANNUAL MEETING AND EXPOSITION 29 October 2006, XP0055051413**

**Description**

**MONOLITHIC DRUG DELIVERY SYSTEM**

**FIELD OF THE INVENTION**

The field of this invention is the application of salting-out and crosslinking of polymers, preferably polyesters to modifying the physicochemical and physicomechanical properties of the said polymers and achieving rate modulated drug delivery system.

**BACKGROUND TO THE INVENTION**

The correlation between the physicochemical and physicomechanical modifications of polymeric materials as well as the related release kinetics from drug delivery devices is significant to our understanding and elucidation of the mechanisms by which phenomena such as salting-out and crosslinking occur (Dashevsky, et al., 2005; Dayal et al., 2005; Huang et al., 2005; Jones et al., 2005; Young et al., 2005).

Salting-out and cross-linking have major implications on the transitions of the physicochemical and physicomechanical properties of polymers that impact on the release kinetics of drug delivery devices and phenomena such as diffusion, relaxation and erosion. (Avgoustakis, 2004; Izutsu and Aoyagi, 2005). The alteration of the three-dimensional polymeric network that results from changes in bond vibrations, morphology, resilience and glass-transition temperature can be attained through ionic interactions between polymer-salt and polymer-polymer during salting-out and crosslinking (Gao et al., 2006).

Salting-out, a colloidal phenomenon, has the capacity to change the morphology, resilience and glass-transition temperature of polymers, by means of salts that cause stochastic fluctuations of the free energy proportional to the salt concentration (Horvath, 1985; Tanaka and Takahashi, 2000). Zhang et al. (1995) showed that the salts can also modulate the release and swelling kinetics of bioerodible polyesters. Pillay and Fassihi (1999) reported on how electrolyte inclusions can alter the configuration and the micro-environment within hydrating matrices to control their swelling kinetics as well as physical rigidity. Furthermore, Hiroshu (2003) described the complexation of divalent ions such as calcium and magnesium to polyesters by ion-dipole bonds. The presence of these ionizable salts allows for non-collapsible diffusion channels to form within the polymeric structure. As the matrix hydrates, the salts and polymer compete for water of hydration, resulting in a programmed release rate (Pillay and Fassihi, 2001, Swenson 2001). Thus the salts will attract water molecules in an effort to solvate themselves, thereby dehydrating the polymer.

One of the principal mechanisms of salting-out is the salt-induced surface tension increase of the water molecules (Eigen and Wicke, 1964; Melander and Horváth, 1977). Electron donor/acceptor interactions are a significant part of the salting-out technique, since the various anionic and cationic species in aqueous solution order certain extents of changes according to their efficacies in salting-out thermoplastic polymers such as OH polyester. Thermodynamic studies by Arakawa and Timashef (1982) demonstrated that the salts that decrease dissolution of hydrophobic polymers are preferentially excluded from the vicinity, strongly bind to the polymers and are called kosmotropes, whereas salts that increase the polymer solubility display weak preferential binding with the polymer and tend to settle at the polymeric surfaces (Galinski et al., 1997; Moelbert et al., 2004).

Although there is a large variety of forces present, including electrostatic and Lifshitz-Van der Waals, the interactions responsible for the salting-out phenomena seem to be dominated by the hydration forces ruled by electron donor/acceptor. Kosmotropes tend to tighten the inter- and intra-molecular structure allowing polymeric interactions, thus enhancing the polymeric properties that include resilience, energy of absorption and the deformability modulus of the polymer.

In aqueous polymeric solutions, salting-out creates stabilization of the water structure, thereby decreasing the hydrogen-bonding between water molecules and the polymeric chain. This is an alternate mode that enhances the hydrophobic interaction between polymeric chains (Bolen and Baskakov, 2001; Valery et al., 2004). These chains are rendered stiff by the introduction of chemical bonds between their monomers (crosslinking), and between the polymeric chains and the salts, which further transform the properties of the polymer (Nystrom et al., 1995). The resulting crosslinked polymeric networks are dimensionally stable, with minimal hydrolysis of the polymer bonds, and exhibit superior structural integrity, making them suitable for sustained drug delivery applications. While polymeric strengths are controlled by the degree of crosslinking, the degradation rate of these networks can be controlled independently by the chemical composition.

Furthermore, in addition to the potential transitions of the polymeric properties, the drug release kinetics and mechanisms may also be significantly influenced by a change in the physicomechanical properties of the polymeric material. Various studies have reported on the mechanisms of drug release from monolithic polymeric devices. In principle, a monolithic device is a simple drug delivery system comprising homogenous drug dispersed within a polymeric matrix. Langer and Peppas (1981) proposed that during the overall release of drugs from monolithic matrices, two distinctive processes could be observed, namely, swelling and 'true' dissolution of the polymer. In case of a swellable system, the device will immediately swell once in contact with the dissolution media. Thus the drug release is controlled by the hydration rate of the system. In order to minimize an initial rapid drug release phase, the polymer employed must be able to form a 'protective' gel layer prior to dissolution. Designing a monolithic system for providing controlled drug release kinetics for water-soluble drugs such as, melatonin, is often a challenge. Pillay and Fassihi, 2000, postulated that these drawbacks may be attributed to the following factors, such as:

(i) The increased hydrophilicity of the drug that causes a burst effect during drug release;
(ii) The lack of accurate management of polymer relaxation or disentanglement over time-dependent processes in relation to drug dissolution and diffusion; and
(iii) The complexity of controlling the increase in the diffusional pathlength with time is not easily attainable (Pillay and Fassihi).

The inherent ineffectiveness of this system can however, be manipulated through the use of salts to modulate the internal geometry of the system. Salts have been highly successful in controlling dissolution and drug release, by demonstrating differential swelling boundaries and texturally variable matrices that manifest as 'peripheral matrix stiffening', a phenomenon that retards the release of watersoluble drugs Additional prior art includes WEBBER, W. L. ET AL: "Characterization of soluble , salt loaded, degradable PLGA films and their release of tetracycline", JOURNAL OF BIOMEDICAL MATERIALS RESEARCH PART A, vol. 41, no. 1, 6 December 1998 (1998-12-06) WEBBER, W. L. ET AL: "Characterization of soluble , salt loaded, degradable PLGA films and their release of tetracycline"JOURNAL OF BIOMEDICAL MATERIALS RESEARCH PART A, vol. 41, no. 1, 6 December 1998 (1998-12-06), pages 18-29, pages 18-29. Therefore, in this work, we evaluated the physicochemical and physicomechanical transitions occurring within salted-out polylactic-co-glycolic acid (PLGA), an $\alpha$-OH polyester, using a statistical approach to develop a mechanistic understanding of its ability to control the release of melatonin from a monolithic drug delivery matrix. These salted-out complexes were termed 'PLGA scaffolds'.

## OBJECT OF THE INVENTION

It is an object of this invention to provide a means for modulating drug delivery through the salting-out and crosslinking of polymers carrying a pharmaceutically active ingredient and to provide a monolithic drug delivery dosage form.

## SUMMARY OF THE INVENTION

In accordance with this invention there is provided a monolithic drug delivery dosage form comprising a salted-out and crosslinked polymer and a pharmaceutically active agent disposed therewith, the salted-out and crosslinked polymer functioning to polymerically entangle the pharmaceutically active agent but, progressively relax on contact with an aqueous medium in use to release the pharmaceutically active agent at a predetermined rate.
There is also provided for the salted-out and crosslinked polymeric material to be poly-lactic co-glycolic acid that is able to control, in use, the release of a pharmaceutically active agent over a prolonged period of time depending on the rate of polymeric relaxation of the polymer on exposure to an aqueous medium.
There is also provided for the salting-out and crosslinking reaction to occur either in combination with the pharmaceutically active agent alternatively with the polymeric material on its own to cause stochastic fluctuations of the reaction which results in polymeric entanglement of the pharmaceutically active agent.
There is further provided for polymeric relaxation reaction to occur in a time dependent manner from the outer boundaries of the dosage form towards its inner boundaries and thus limit outward diffusion of the entangled pharmaceutically active agent in a controlled fashion as the inward ingress of aqueous medium causes a progressive relaxation of the polymeric chains from the outer boundaries of the dosage form or tablet in a direction towards its inner core.
There is also provided for the salting-out and crosslinking reaction of the polymer and a crosslinking reagent, preferably an inorganic salt further preferably an inorganic ionic salt, preferably from the Hofmeister series of salts examples of which are sodium chloride, aluminium chloride and calcium chloride.
There is further provided for the polymer to be a polyester, preferably a polylactic acid and/or its co-polymers and further preferably poly-lactic co-glycolic acid.
The invention extends to a method of producing a monolithic drug delivery dosage form comprising a salted-out and crosslinked polymer and a pharmaceutically active agent disposed therewith comprising salting-out or crosslinking a polymer to polymerically entangle the pharmaceutically active agent but, progressively relax on contact with an aqueous medium in use to release the pharmaceutically active agent at a predetermined rate.

There is also provided for the salted-out and crosslinked polymeric material to be poly-lactic co-glycolic acid that is able to control, in use, the release of a pharmaceutically active agent over a prolonged period of time depending on the rate of polymeric relaxation of the polymer on exposure to an aqueous medium.

There is also provided for the salting-out and crosslinking reaction to occur either in combination with the pharmaceutically active agent alternatively with the polymeric material on its own to cause stochastic fluctuations of the reaction which results in polymeric entanglement of the pharmaceutically active agent.

There is also provided for the salting-out and crosslinking reaction of the polymer and a crosslinking reagent, preferably an inorganic salt further preferably an inorganic ionic salt, preferably from the Hofmeister series of salts examples of which are sodium chloride, aluminium chloride and calcium chloride.

There is further provided for the polymer to be a polyester, preferably a polylactic acid and/or its co-polymers and further preferably poly-lactic co-glycolic acid.

## BRIEF DESCRIPTION OF AN EMBODIMENT OF THE INVENTION

One embodiment of a monolithic drug delivery dosage form according to the invention will be described below with reference to the accompanying figures in which:

Figure 1    illustrates typical Force-Distance and Force-Time profiles of PLGA scaffolds for determining (a) energy absorbed (b) deformability modulus and (c) matrix resilience (N=10);

Figure 2    is a series of selected scanning electron micrographs of PLGA scaffolds demonstrating the surface morphology of PLGA: salted-out with NaCl [(a) and (b)], $CaCl_2$, [(c) and( d)], and $AlCl_3$ [(e) and (f)]:

Figure 3    shows profiles depicting differences in the physicomechanical properties of PLGA scaffolds. Plot (a) Resilience (R), (b) Energy absorbed (E) and (c) Deformability modulus (DM);

Figure 4    shows typical surface response plots depicting the effects of the independent formulation variables on the physicomechanical properties of the salted-out PLGA scaffolds, namely (a) Resilience (R) (%); (b) Energy absorbed (E) (J); and (c) Deformability modulus (DM) (N/mm). *AC= A/Cl$_3$, CC=* $CaCl_2$*, SC= NaCl. -1=0%$^w$/$_v$ 0=5%$^w$/$_v$ 1=10%$^w$/$_v$;*

Figure 5    presents a series of typical profiles used to construe the main and interactions effects of NaCl (a) and (d); $CaCl_2$ (b) and (e); $AlCl_3$ (c) and (f) for resilience in the presence of a combination of parameters;

Figure 6    illustrates profiles demonstrating the correlation between experimental and fitted response values. R=Resilience; E=Energy absorbed; DM= Deformability modulus;

Figure 7    shows five superimposed FTIR profiles depicting transitions from native PLGA to salted-out PLGA scaffolds. (a) = Native PLGA, (b)-(e) = PLGA salted-out with NaCl, $CaCl_2$, $AlCl_3$ and a combination of NaCl + $CaCl_2$+ $AlCl_3$ respectively;

Figure 8    illustrates Differential Scanning Calorimetry (DSC) profiles of native and salted-out PLGA scaffolds demonstrating thermal transitions of (a) native PLGA, (b) PLGA salted-out with NaCl, (c) $CaCl_2$, (d) $AlCl_3$, and (e) a combination of NaCl, $CaCl_2$, and $AlCl_3$; and

Figure 9    shows release profiles of melatonin from polymer when drug was either (a)(b) non-crosslinked or (c) crosslinked during the formation of PLGA scaffolds.

## DETAILED DESCRIPTION OF AN EMBODIMENT OF THE INVENTION

### 1. Materials and Methods

#### *1.1 Materials*

PLGA was obtained from Boehringer Ingelheim Pharma (Ingelheim, Germany) (Resomer® RG504 50:50; $M_w$ 48,000; i.v. 0.48-0.60dl/g). Acetone was used as a solvent, and analytical grades of sodium chloride (NaCl), calcium chloride ($CaCl_2$), (Rochelle Chemicals, South Africa) and aluminium chloride ($AlCl_3$) (Merck, Darmstadt, Germany) were used as the ionic salts. Disodium hydrogen crthophosphate, ($Na_2HPO_4$), and potassium dihydrogen phosphate ($KH_2PO_4$)

were obtained from Saarchem (Pty) Ltd., South Africa. Model drug, melatonin was obtained from Sigma-Aldrich Co., Germany.

### 1.2 Building the experimental design

A 3 factor, 3 level Box-Behnken statistical design was built in order to model the number of experiments needed for formulation optimisation and to establish the main and interaction effects of the independent formulation variables on the physicochemical and physicomechanical properties of the PLGA scaffolds using Minitab V14 (Minitab, USA).

### 1.3 Preparation of PLGA scaffolds

PLGA scaffolds were prepared by salting-out and subsequent crosslinking using a combination of acetone and ionic salts in accordance with a Box-Behnken design template outlined in Table 1. Fourteen polymeric solutions comprising 0.4g of PLGA dissolved in 15mL of acetone were prepared. The crosslinking solutions comprising 75mL of $0^w/_v$, $5^w/_v$ or 10%$^w/_v$ of NaCl, $CaCl_2$ or $AlCl_3$ was added to the polymeric solution and agitated for a period of 30 minutes. The resultant PLGA scaffolds were removed from the crosslinking solution washed thrice with 500mL deionized water and dried to constant mass at room temperature. Each PLGA scaffold was stored for a maximum of 48 hours prior to physicochemical and physicomechanical evaluation.

**Table 1:** Box-Behnken design template with randomly generated PLGA Scaffold formulations

| Formulations | Randomized Run Order | [NaCl] (%$^w/_v$) | [CaCl$_2$] (%$^w/_v$) | [AlCl$_3$] (%$^w/_v$) |
|---|---|---|---|---|
| 1 | 6 | 10 | 5 | 5 |
| 2 | 11 | 5 | 10 | 0 |
| 3 | 7 | 5 | 5 | 5 |
| 4 | 4 | 5 | 0 | 10 |
| 5 | 2 | 10 | 5 | 10 |
| 6 | 3 | 5 | 10 | 0 |
| 7 | 5 | 5 | 5 | 5 |
| 8 | 12 | 10 | 5 | 10 |
| 9 | 8 | 5 | 10 | 0 |
| 10 | 13 | 5 | 5 | 5 |
| 11 | 9 | 5 | 0 | 10 |
| 12 | 14 | 10 | 5 | 10 |
| 13 | 1 | 5 | 10 | 0 |
| 14 | 10 | 5 | 5 | 5 |

The quadratic model for the responses is shown in Equation 1:

$$Response = b_0 + b_1 [NaCl] + b_2[CaCl_2] + b_3[AlCl_3] + b_4[NaCl]^2 + b_5 [NaCl][CaCl_2] + b_6[NaCl][AlCl_3] + b_7[CaCl_2][AlCl_3] + b_8[CaCl_2]^2 + b_9[AlCl_3]^2 \qquad \text{(Equation 1)}$$

Where, the *Response* is associated with each factor level, $b_{0...}b_9$ are the regression coefficients, and [NaCl], [CaCl$_2$] and [AlCl$_3$] are the independent formulation variables.

### 1.4 Morphological characterization of the PLGA scaffolds

The surface morphology of the PLGA scaffolds was assessed from Scanning Electronic Microscopic (SEM) images employing a thermal emission JEOL JSM-(Japanese Electronic Optical Laboratories, Tokyo, Japan) electron micrograph. Samples of PLGA scaffolds were sectioned and mounted on aluminium stubs prior to sputter-coating with a layer of carbon. Each sample was viewed under varying magnifications at an accelerating voltage of 20 kV.

*1.5 Determination of the physicomechanical properties of the PLGA scaffolds*

The physicomechanical properties of the PLGA scaffolds were evaluated using a Texture Analyzer (TA.*XTplus* Texture Analyzer, Stable Microsystems, UK). Stress-strain profiles with a high degree of accuracy and reproducibility were capture at a rate of 200 points per second employing Texture Exponent software V3.2 and subsequently analyzed. A 3.5cm flat-tipped circular steel probe was attached to the force transducer. The parameter settings employed to obtain the energy absorbed, deformability modulus and matrix resilience are shown in Table 2.

**Table 2:** Textural parameter settings

| Settings | Energy absorbed and Deformability modulus | Matrix resilience |
|---|---|---|
| Pre-test speed | 1mm/sec | 1 mm/sec |
| Test speed | 0.5mm/sec | 0.5mm/sec |
| Post-test speed | 1mm/sec | 1 mm/sec |
| Compression force / strain | 40N | 50% |
| Trigger type | Auto | Auto |
| Trigger force | 0.5N | 0.5N |
| Load cell | 50kg | 50kg |
| Distance | 20mm | 20mm |

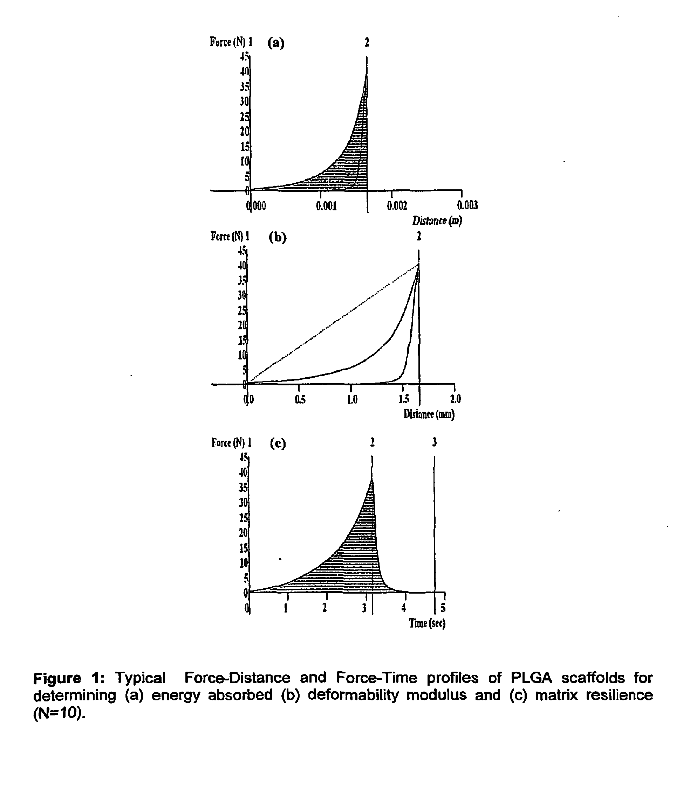

To elucidate the energy absorbed, matrix resilience and deformability modulus, Force-Distance and Force-Time profiles for each PLGA scaffold was generated. Typical textural profiles used for quantifying the physicomechanical properties are depicted in Figure 1.

Figure 1 (a) depicts the anchors used in a Force-Distance profile for calculating the energy absorbed i.e. the total area under the curve (AUC) [Nm=Joules] between anchors 1 and 2. Figure 1(b) depicts the anchors employed for determining the Deformability modulus (the tendency of the PLGA scaffolds to change shape upon the application of stress) i.e. the gradient between anchor 1 and the maximum force attained during sample analysis. Figure 1(c) depicts the anchors employed for calculating the matrix resilience, i.e. the ratio of the AUC between anchors 2 and 3 and 1 and 2 ($AUC_{32}/AUC_{12}$) for a Force-Time profile. Note that the resilience may be defined as

*1.6 Elucidation of the molecular structural transformations within PLGA scaffolds*

Fourier Transform Infra-Red (FTIR) spectroscopy was performed on native PLGA and PLGA scaffolds to determine chemical transformations potentially occurring within the polymeric backbone due to salting-out and subsequent crosslinking using a Nicolet Impact 400D (Nicolet Instrument Corporation, Pennsylvania, USA) instrument. The potassium bromide (KBr) disc approach was employed, whereby 7.5mg samples of each PLGA scaffold was triturated with 200mg of KBr and compressed in a transparent circular disc using a Beckman Hydraulic Press (WIKA Instruments (Pty) Ltd, Johannesburg, South Africa). Background scans were obtained for all samples and the % transmittance was recorded between 4000 - 400cm$^{-1}$ at an intermediate resolution.

*1.7 Thermal transition analysis of the PLGA scaffolds*

Differential Scanning Calorimetry (DSC) was used to record transitions in specific heat capacity and latent heat, which indicated changes in the amorphous or crystalline structure as a result of scaffold formation from crosslinking native PLGA. Thermal transitions were recorded on a Perkin-Elmer Pyris-1 connected to a controller model TAC1/DX (Perkin-Elmer, Inc. USA). Samples were heated in increments from 25°C to 400°C at a rate of 10°C/min. Samples of 5-10mg of each PLGA scaffold was placed within a crimped aluminium pan and subjected to the heat gradient. Thermograms were obtained and subsequently analyzed.

*1.8 Preparation of the salted-out PLGA monolithic matrix*

Formulations of either drug-free PLGA or drug-loaded PLGA samples were salted-out at various concentrations in accordance with a Box-Behnken statistical design. Matrices were prepared by direct compression of a mixture comprising 300mg salted-out PLGA and 10mg melatonin for the drug-free PLGA and 350mg of each drug-loaded variant was compressed using a Beckman Hydraulic Press (Beckman Instruments, Inc., Fullerton, USA).

*1.9 Drug entrapment efficiency (DEE) of the PLGA scaffolds*

DEE studies were performed by immersing each scaffold in 100mL acetone to effect complete dissolution of the scaffold. Thereafter, melatonin content was established in triplicate using UV-spectroscopy at 278nm.

*1.10 In vitro drug release from the monolithic matrices*

Drug release studies were conducted in 500mL phosphate buffered saline (PBS) (pH 7.4; $37^0$C) using a modified USP25 apparatus at 50 rpm. Melatonin assays were performed with UV-spectroscopy (278 nm) (SPECORD 40, Jena, Germany). The dissolution data was subjected to a model-independent analysis known as the time-point approach. Briefly, the mean dissolution time set at 30 days ($MDT_{30}$) for each formulation was calculated. The application of the mean dissolution time provided a more precise analysis of the drug release performance and a more accurate comparison of several dissolution data sets. Equation 2 was employed in this regard:

$$M \ D \ T \ = \ \sum_{i=1}^{n} t_i \ \frac{M_t}{M_\infty} \qquad \text{(Equation 2)}$$

Where $M_t$ is the fraction of dose released in time $t_i = (t_i + t_{i-1}) / 2$ and M. corresponds to the loading dose.

## 2. Results and Discussion

### 2.1 Proposed interactions between native PLGA polymeric chains and crosslinking ions

The solvated ion pairs of $Na^+$, $Ca^{2+}$, and $Al^{3+}$ develop into electron nodes that facilitate ionic reactions. These solvated ion pairs are able to attract the adjacent cations of $Cl^-$ and $O^{2-}$ within the polymeric matrix thus contributing to crosslinking of lactide and glycolide chains within the PLGA molecular structure. This crosslinking reaction depends primarily on the ionization energies of the salting-out ion, hydration enthalpies in solution and the thermodynamic stability of the monomeric PLGA units. Furthermore, the coordination number of each salt, 8, 6 and 4 for $Al^{3+}$, $Na^+$ and $Ca^{2+}$ respectively and atomic size of ions (Table 3) also influences the attraction of adjacent cations during crosslinking with the ion and/or salt possessing the highest coordination number and atomic size having the most influence on the crosslinking reaction and subsequently contributes a central factor in modifying the native PLGA polymeric structure to produce a robust PLGA scaffold.

**Table 3:** Physicochemical properties of the salts employed during crosslinking of native PLGA

| Salt Type | Atomic radius of Metals (pm) | Metal Coordination Number | Cation Coordination Number |
|---|---|---|---|
| NaCl | 186 | 6 | 6 |
| $CaCl_2$ | 197 | 4.2+/-0.5 | 5.4+/- 0.3 |
| $AlCl_3$ | 125 | 8 | 6 |

The salts used in this study, namely NaCl, $CaCl_2$ and $AlCl_3$ differ with regard to the physicochemical, physicomechanical and morphological structure of the resultant PLGA scaffolds. Furthermore, the shape and stereo-orientation within the PLGA structure differs and therefore, the ability of water molecules to be imbibed within the matrix depends on the rate of polymer-salt interactions, the reactivity, atomic size and coordination number of the concerned ions, the nature of the crystal-lattice packing of ions and the polymeric substrate present during salting-out and subsequent crosslinking.

### 2.2 Scanning electron microscopic image analysis of the PLGA scaffold morphology

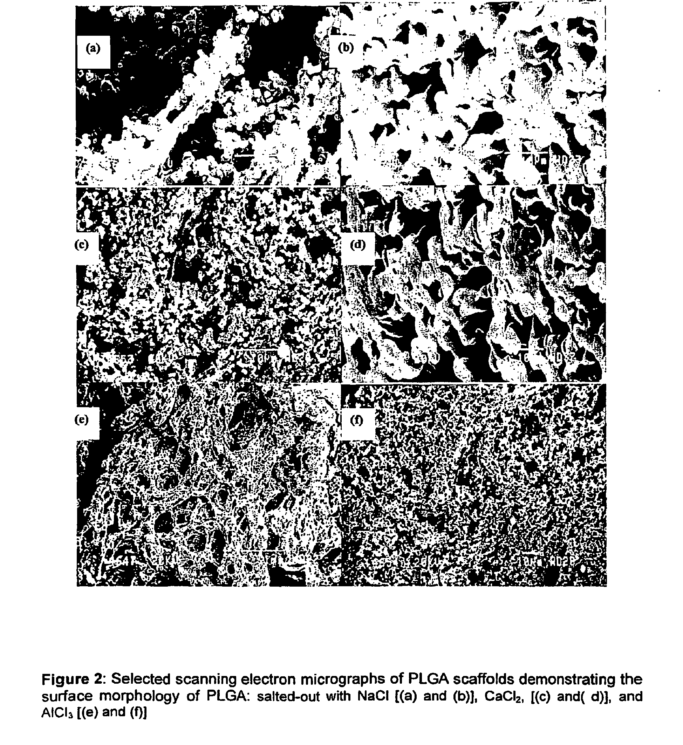

Three-dimensional architecture comprising various fiber volume and diameters, interconnections and pore sizes were obtained from polymer-salt interactions as a result of crosslinking during PLGA scaffold formation. The presence of salts generated areas of high entropy at the solid-liquid interfaces resulting in altered fibrous structures with distinct morphologies (Figure 2). The ionic interactions between the salts and PLGA molecules were dependent on the ionization energies of crosslinking ions, hydration enthalpy in solution as well as the thermodynamic stability and molecular accumulation of salts and water at the lactide-glycolide strands of native PLGA. As a result several morphological conformations of each PLGA scaffold were obtained (Figure 2). Furthermore, the coordination number of each salt influenced the number of covalent bonds formed. Hence, hydrogen bonding and other intra and inter-ionic forces located on the PLGA molecule (oxygen residues) cluster-packed with water molecules decided the nature and size of pores and fibers of the newly formed PLGA scaffolds.

SEM images of PLGA scaffolds salted-out with NaCl revealed a uniform distribution of interconnected pores, divided by struts of microporous structures that maintained homogeneity of crosslinked fibers in a neuronal meshwork archetype (Figures 2a and 2b). These fibers possessed pores and fiber diameters ranging between 0.1-1.4$\mu$m, and fiber volumes ranging from 0.01-0.03$\mu$m$^3$. In Figures 2c and 2d, the divalent salt CaCl$_2$ produced a fibrillar composite PLGA scaffold with interconnecting channels in a distinct voluminous trabecular formation with scales of pore sizes and diameters ranging between 7.5-15$\mu$m and fiber volumes of 800-1400$\mu$m$^3$. In Figures 2e and 2f, PLGA scaffolds salted-out with a trivalent salt namely AlCl$_3$ revealed fine fibrous morphologies with distinct crosslinks that resulted in a ramified interconnection of the PLGA scaffold design with pore sizes and diameters ranging from 0.03-0.10$\mu$m and fiber volumes between 0.09-0.17$\mu$m$^3$.

In general, introduction of salting-out ions to the polymeric solution facilitated the native PLGA polymeric structure to assume a fixed three-dimensional configuration into crosslinked lactide-glycolide chains. Furthermore, the adjacent voids resulting from such a configuration were able to accommodate water molecules in accordance to the dimensions of voids created due to short and long distance interactions between native PLGA chains. These voids significantly provided the space for binding water molecules which was dependent on the rate of the salting-out and subsequent crosslinking reaction. An increase in the fibrillar nature of the PLGA scaffolds augmented the physicomechanical properties such as matrix resilience. The distinct differences in morphology revealed in each micrograph of the PLGA scaffolds suggest the versatility of the scaffold and hence may be suitable for tailored manufacturing that match specific applications.

### 2.3 Textural profile analysis to quantify the physicomechanical properties of PLGA scaffolds

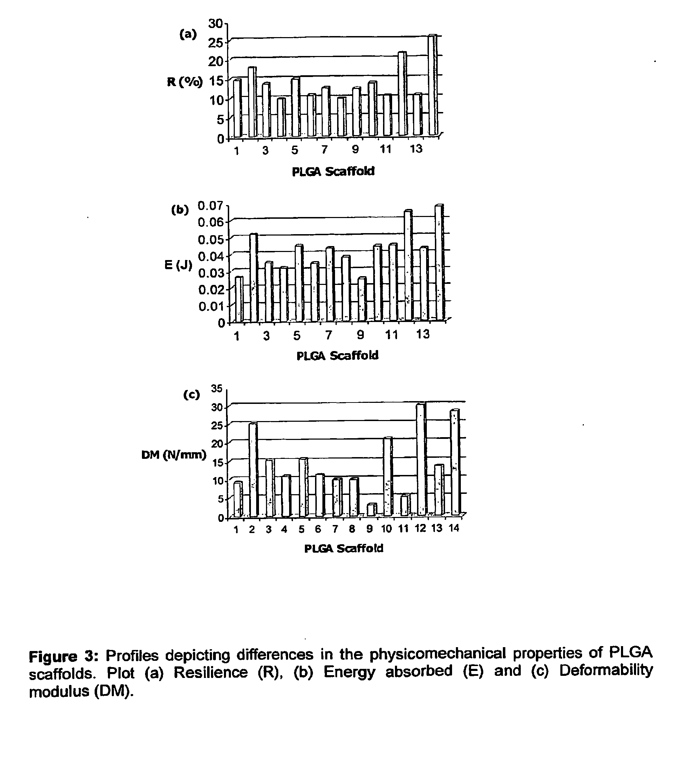

Analysis of the textural profiles provided an insight on the Stress-Strain relationships for the PLGA scaffolds. Results demonstrated that native PLGA could be modified into a highly resilient polymeric material by rapid ionic salting-out and subsequent crosslinking in order to achieve elasticity that depended mainly on the type and the concentration of salt employed. The matrix resilience values of PLGA scaffolds salted-out with NaCl and AlCl$_3$ were superior to that of PLGA scaffolds salted-out with CaCl$_2$ (Figure 3).

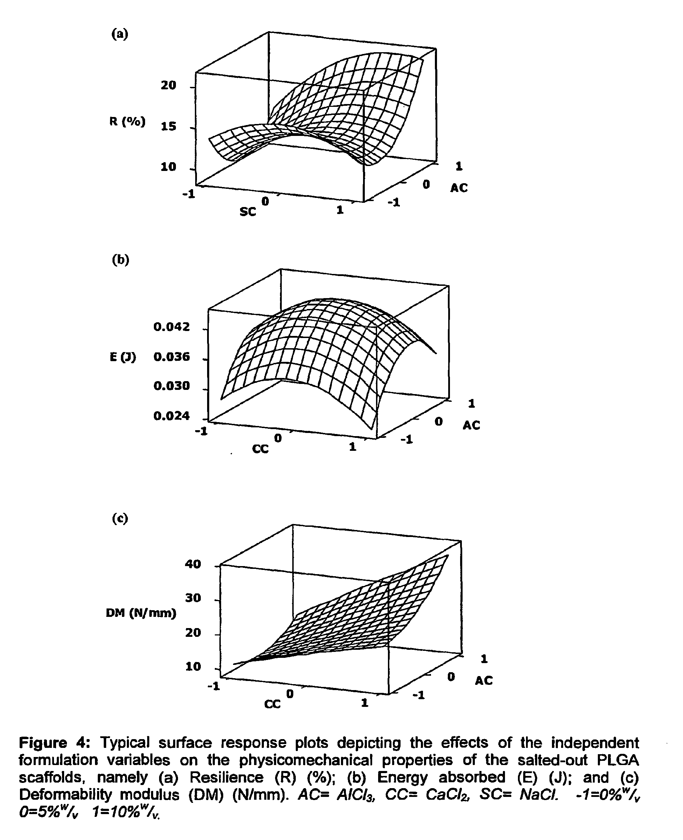

The physicochemical nature of the salt employed during salting-out created a micro-environment which accentuated the viscoelastic behaviour of the PLGA scaffold. The viscoelasticity caused densification of the PLGA scaffolds, which resulted in resistance of the scaffold to deform under stress during textural analysis. Figures 4a and 4b revealed significant increases in the resilience and energy absorbed when concentrations of NaCl and AlCl$_3$ were increased, for instance in formulations 2, 12 and 14. The concentration of CaCl$_2$ was found to be inversely proportional to the energy absorbed as demonstrated in formulations 6, 9 and 13. The increase in resilience and deformability moduli was linearly correlated to the quantity of energy absorbed by the PLGA scaffolds per unit volume shown in Figure 4b and c.

The modification of the physicomechanical properties revealed by these profiles is consistent with the dense surface morphology of the PLGA scaffolds depicted in Figure 2. In general, salts that produced more compact polymeric structures with smaller and more uniform pores, such as NaCl and AlCl$_3$ accentuated the physicomechanical properties of the PLGA scaffolds, whereas salts that produced larger pores decreased the resilience.

Furthermore, the resultant accumulation of additional water molecules conferred more dipoles to the matrix, which were less responsive to activity within the PLGA backbone. Ferry (1980) reported that the presence of water molecules which are dipole and contributors of interfacial tension decreases matrix resilience. The proximity of the polarising dipoles to the PLGA backbone and the extent to which they are influenced by the configurational activity of the PLGA chain directly influenced the total resilience, energy absorbed, and deformability modulus of the PLGA scaffold.

### 2.4 Response surface plots indicating interaction between dependent variables

Surface plots (Figure 4) were constructed to visually demonstrate the individual and synergistic effects of the salts on modifying the physicomechanical properties of native PLGA by scaffold formation. Figure 4a revealed that at lower concentrations (between 0-5%$^w$/$_v$), NaCl significantly increased resilience of PLGA scaffolds up to a limit of 15% at which any further increase of NaCl (above 5%$^w$/$_v$) resulted in a decreased resilience. At low concentrations (between 0 and 5%$^w$/$_v$) of AlCl$_3$ a resilience of 10% was maintained and a further increase in AlCl$_3$ beyond 5%$^w$/$_v$ resulted in a linear increase of resilience.

Figure 4b demonstrated that a concentration of CaCl$_2$ above 5%$^w$/$_v$ lowered the energy absorbed and that a 10%$^w$/$_v$ CaCl$_2$ significantly diminished the ability of the PLGA scaffolds to absorb energy. Furthermore, Figure 4c depicts that the concentration of CaCl$_2$ had a minor effect on the deformability modulus of the PLGA scaffolds, whereas an increase in AlCl$_3$ concentrations largely increased the deformability modulus of the PLGA scaffolds. The following three-dimensional surface plots depict each of the responses (physicomechanical properties) resulting from changes in the independent formulation variables.

### 2.5 Determination of the main and interactions effects on the various responses

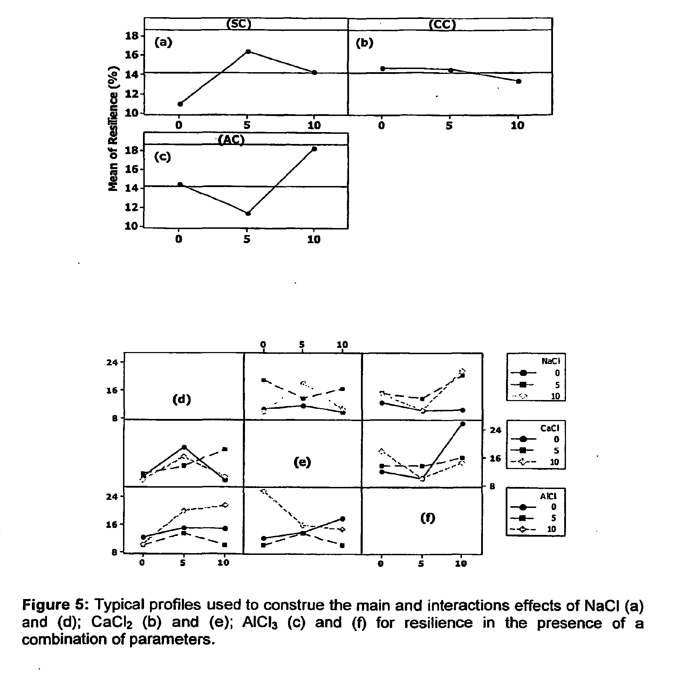

The main and interaction effects of the salt type and concentration and their influence on the physicomechanical properties of PLGA are demonstrated in Figure 5. The plots of main and interaction effects were run to provide a visual authentication of the significant variables on the resilience, energy absorbed, and deformability modulus model terms. The effects on the responses were found to be attributable to the main effects (i.e. the salts) as well as other interactions such as the polymeric substrate, solvent, water volume, and salting-out reaction time up to the last variable interactions. The degree of interactions was observed to rise exponentially with the number of factors. Digression from the centre-point designated a change in response over the tested range. Visually, the discrepancies in the mean values of the plot are the least squares estimate for the effect. Huge discrepancies indicated by higher gradients as in Figures 5a, c, d, and f signified important variables while diminutive discrepancies indicated by lower gradients signified trivial variables in a given plot. Parallel plots as in Figures 5b and 5e implied minimal or no interaction of the independent formulation variable.

As demonstrated in Figure 5, the type and concentration of salt played a vital role in the nature and extent of PLGA modification. In Figure 5c NaCl, a monovalent salt had the greatest effect on resilience with optimal resilience experienced at $5\%^w/_v$. The divalent salt $CaCl_2$ had a minor effect on resilience, regardless of the change in concentrations. It was also observed that the resilience increased with the increase in concentration of the trivalent salt $AlCl_3$ from $5\%^w/_v$. A similar correlation could be seen from Figure 3 and Figure 4. These observed transitions in resilience can be explained as a contribution from a combination of several effects such as variations of the water molecule structure present in the matrix hydration sheath as well as the adjustments of the interactions between the PLGA and solvent due to the presence of various salts.

### 2.6 Correlation between the experimental and fitted responses employing a quadratic model

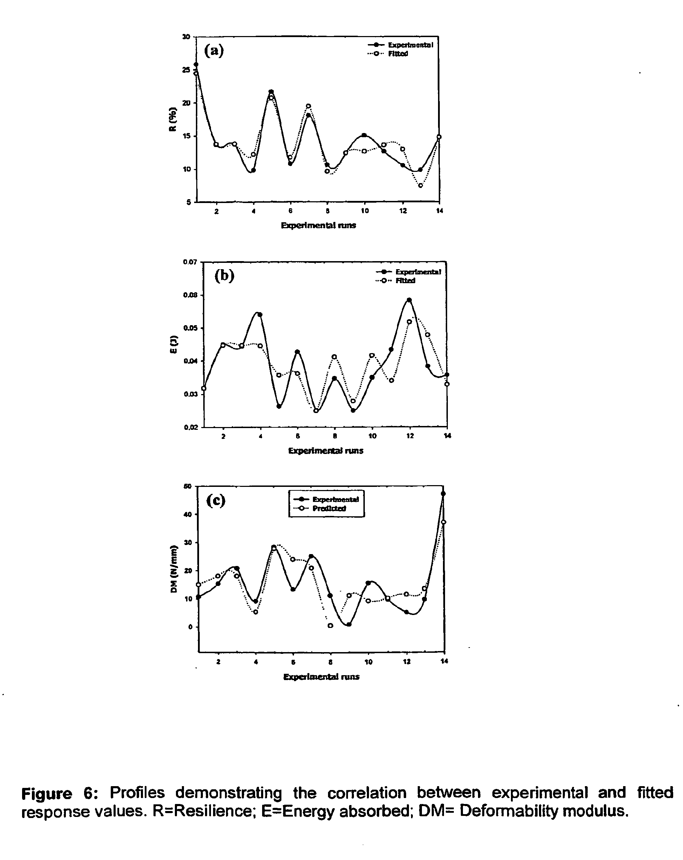

Figure 6 depicts the close correlation between the fitted and experimental values for the dependent formulation variables, namely, resilience, energy absorbed, and deformability modulus. No significant differences were noted between the fitted and experimental values ($p>0.05$). This therefore, indicated that the Box-Behnken design provided a suitable statistical approach to evaluate the effects of various salts on modifying native PLGA into salted-out PLGA scaffolds.

### 2.7 Assessment of the polymer-salt interactions and polymeric structural transitions

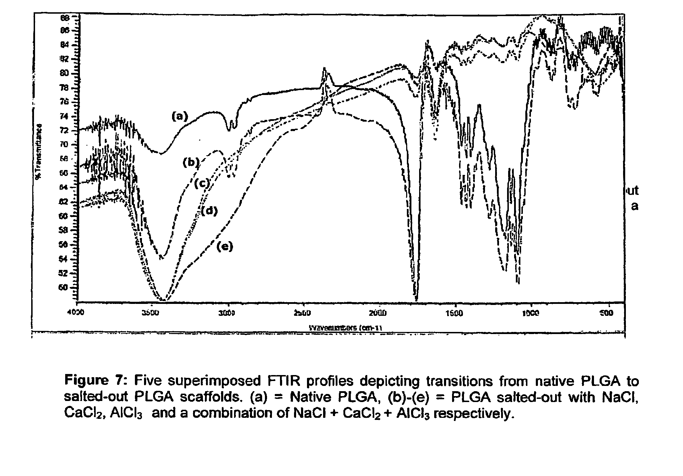

As observed in the FTIR profiles depicted in Figure 7, the functional groups of PLGA involved in interactions with the salts were similar. However, the degree and extent of these bond vibrations at finger-print regions varied. This implied that the polymer-salt interaction in solution was clearly influenced by the molecular structure of the salt as well as the chemical backbone of PLGA that resulted in the diverse morphological, physicochemical, and physicomechanical transitions demonstrated by the PLGA scaffolds.

During salting-out and subsequent crosslinking the salts ionized in water and reacted with the $\delta, \pi, \sigma$, C-O and H-groups, to form hydrogen, ether bonds and salt-oxygen bonds between the PLGA chains. This resulted in crosslinking of the lactide-glycolide units within the PLGA molecular structure. Hydrogen, ether and ion-oxygen bonds were formed by the salts between free pendant carbonyl groups of PLGA into resonance stabilized bonds. This was demonstrated by the prominent vibrational increase in the frequency ranges of 1180-1300 cm$^{-1}$ (COC), 3200-3700 cm$^{-1}$ (OH stretching), 1600-1900 cm$^{-1}$ (CO) and the synchronous decrease in the C=O groups (bending) vibration intensities in the range of 1530-2500 cm$^{-1}$. The intensities of transmittance of aliphatic ester bonds present in PLGA were also decreased. Furthermore, crosslinks formed by the non-uniform length of polymeric chains resulted in a three-dimensional dense network that caused further vibrational intensities (Figure 7).

### 2.8 Thermal transitions within the PLGA scaffolds

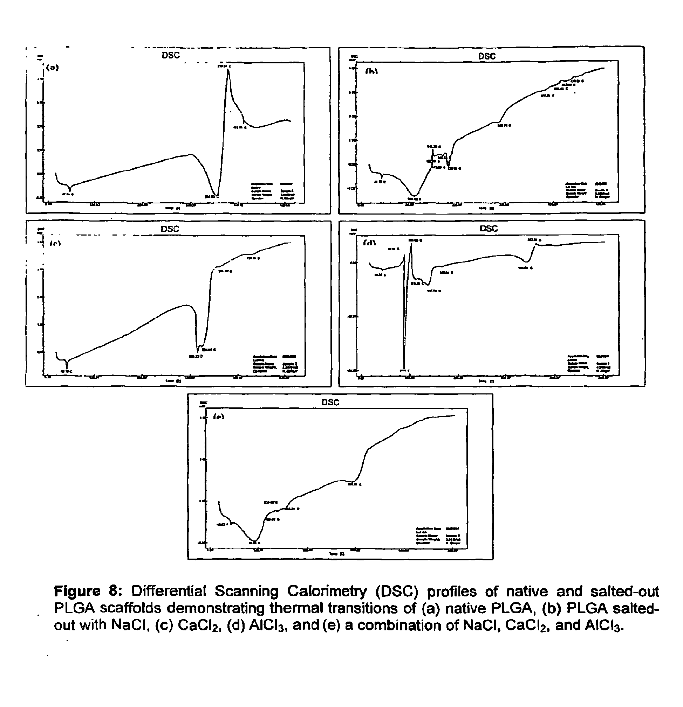

Figure 8 demonstrates the enthalpy changes due to various polymer-salt interactions. During salting-out of PLGA, the enthalpy of the PLGA scaffolds was enhanced by the increase in steric strain attributable to a gain of electron energy. Furthermore, enthalpy changes also occurred as a result of bond formation that increased the bond-energy of the system and resonance stabilization thereby increasing the internal energy and enthalpy of the PLGA scaffolds. Furthermore, the steric strain caused by bond stretching, bond-angle deformation, and polymer-salt interactions increased the internal energy and enthalpy of the system. As molecules gained sufficient mobility to initiate the crosslinking reaction exothermic changes occurred in the temperature range of 40-47°C which essentially described the glass transition point.

Table 4 lists the significant parameters obtained from analysis of the DSC profiles. In Figure 8a-e a step transition from glass to rubbery state on the heating cycle was clearly observed as sharp peaks at 47.24, 41.79, 40.19, 43.35 and 42.82°C respectively. The melting point range of PLGA scaffolds was 140-160°C which was a significant reduction from native PLGA that has a melting point range of 280-300$^0$C. Re-crystallization and further decomposition of the polymeric-salt complex took place between 410-430°C.

**Table 4:** Thermal parameters of native and salted-out PLGA employing DSC

| Formulation | Tg°C | mp°C | Tc°C | Td°C |
|---|---|---|---|---|
| Native PLGA | 47.24 | 280-300 | 354.85 | 411.65 |
| B | 41.79 | 148.30 | 285.61 | 428.05 |
| C | 40.19 | 280 | 315.30 | 426.94 |
| D | 43.35 | 166.54 | 343.04 | 420.06 |

*Tg =glass transition temperature; mp =melting point; Tc =re-crystallization temperature; Td =degradation temperature*

Depending on the type of salt employed, water can be trapped within the polymeric matrix and thus depress the $T_g$. The size of ions ($Al^{3+} < Na^+ < Ca^{2+}$) determined the degree of $T_g$ depression. Kelly and co-workers (1987) reported that a significant change in $T_g$ is observed with a 10% increase or decrease in the water content within the polymeric matrix. Thus the dynamic activity of PLGA chains may be restricted by confines of water molecules within the matrix. The large ionic radius of $Ca^{2+}$ led to an increase in the number of voids within the scaffold utilized by water molecules. Conversely, $Na^+$ and $Al^{3+}$ ions decreased the number of voids. Hence PLGA scaffolds salted-out with $CaCl_2$ had a lower $T_g$ of 40.19°C. Studies by Paulaitis and co-workers (2004) have yielded verification on the dependence of polymeric hydration free energy on the solute size and shape. Moreover, work done by Bemazzani and co-workers (2003) demonstrated that precipitation of polymers from dilute solutions would depress the $T_g$ which may be attributed to the crosslink induced shorter and free chain ends that disarray the crystallinity of the matrix. This was consistent in the findings of this study as well.

### 2.9 Characterization of the in vitro drug release from the monolithic matrices

Theoretically, the primary drug release mechanism from both PLGA monolithic matrices should be diffusion through the matrix layer by a Fickian release mechanism. However, matrix swelling and erosion also played a significant role. Since melatonin is water-soluble and PLGA is a hydrophobic polymer, the rate of drug release decreased as a function of time as the diffusional path length for drug release increased over time when the dissolution medium front approached the center of the matrices.

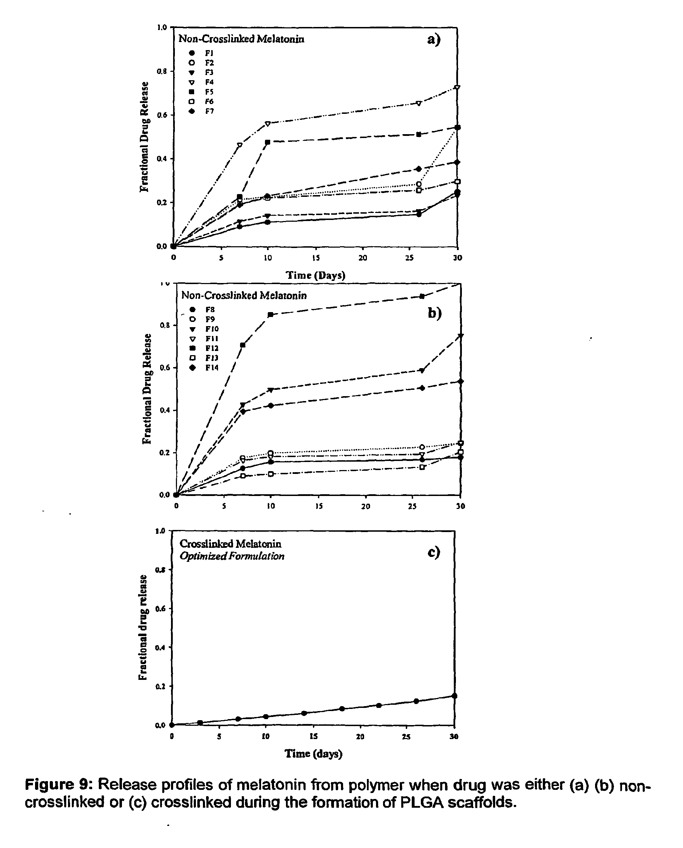

When melatonin was incorporated in a non-crosslinked manner, DEE varied between 46-90%. On the other hand, when melatonin was involved in the crosslinking process, an average DEE of 90% was achieved. Release profiles revealed that the monolithic matrices prepared by salting-out and subsequently crosslinking PLGA with melatonin employing various salts were able to achieve zero-order release kinetics with less than 20% melatonin released over a period of 30 days (Figure 9c). Monolithic matrices demonstrated a mean dissolution time at 30 days ($MDT_{30}$) of 6 to 26 (Figure 9a and b). The fractional drug release ($M_t/M_\infty$), and the drug release kinetics were calculated using the power law $M_t/M_\infty = k_0 t$, where k, the kinetic constant was found to be $k_0$ of 0.004 to 0.038. The optimized formulation demonstrated 30-day zero-order kinetics for *in vitro* melatonin release (Figure 9c). This study demonstrated that crosslinking significantly controlled the rate of drug release due to the strong interactions between the drug and polymeric chains. The slow diffusion of melatonin from salted-out PLGA occurred due to shielding of polymeric reaction sites and coiling which prevented the maintenance of the same effective collision rate at which chemical reactions are obtained between the same functional groups and the aqueous environment in non crosslinked polymer molecules, thus conferring the ability to achieve ideal zero-order drug release.

### 3. Conclusion

The salting-out and subsequent crosslinking approaches applied in the study in order to modify the physicochemical and the physicomechanical properties of native PLGA and achieve more controlled drug release kinetics displayed significant potential. The resulting crosslinked PLGA scaffolds exhibited superior structural integrity as determined by parameters such as resilience, energy of absorption and deformability moduli which contributed to the overall robustness and level of porosity of the PLGA scaffolds making it a favourable candidate for controlled drug delivery. The close correlation between the experimental and fitted response values demonstrated the reliability of the selected statistical design for experimental optimisation. The monovalent, divalent and trivalent ionic salts employed in the study proved to be suitable in transforming the structure of native PLGA into a modified PLGA scaffold with superior physicomechanical properties. These superior properties were confirmed by textural profile analysis, SEM, DSC and FTIR studies. In general, the degree of bond formation in the PLGA backbone demonstrated by vibrational intensity transitions from FTIR studies, in combination with the newly formed hydrolytically degradable PLGA crosslinks present a possible application of the PLGA scaffolds in rate-modulated drug delivery. This study has also demonstrated that salting-out and subsequent crosslinking of PLGA can significantly control the rate of drug release as a result of strong bonds formed between the drug and PLGA during crosslinking ultimately leading to zero-order release kinetics.

## 4. References

1. Avgoustakis K. Pegylated poly(lactide) and poly(lactide-co-glycolide) nanoparticles: preparation, properties and possible applications in drug delivery. Curr Drug Delivery, 2004;1(4):321-33.

2. Arakawa T, Timasheff SN, Mechanism of poly(ethylene glycol) interaction with proteins, Biochemistry 24 (1985) 6756- 6762.

3. Asthagiri D, Pratt LR, Paulaitis ME, and Rempe SB. Hydration Structure and Free Energy of Biomolecularly Specific Aqueous Dications, Including $Zn2+$ and First Transition Row Metals. J. Am. Chem. Soc.2004; 126(4):1285 - 1289.

4. Bolen DW, Baskakov IV. The osmophobic effect: natural selection of a thermodynamic force in protein folding, J. Mol. Biol. 310 (2001) 955-963.

5. Dayal P, Pillay V, Babu RJ, Singh M. Box-Behnken experimental design in the development of a nasal drug delivery system of model drug hydroxyurea: characterization of viscosity, in vitro drug release, droplet size, and dynamic surface tension. AAPS PharmSciTech. 2005;6(4):E573-85.

6. Dashevsky A, WagnerK, Kolter K, Bodmeier R. Physicochemical and release properties of pellets coated with Kollicoat SR 30 D, a new aqueous polyvinyl acetate dispersion for extended release. Int J Pharm. 2005 16;290(1-2):15-23.

7. Galinski EA, Stein M, Amendt B, Kinder M. The kosmotropic (structure-forming) effect of compensatory solutes, Comp. Biochem. Physiol. 117A (1997) 357 - 365.

8. Gao Q, Xue S, Doneanu CE, Shaffer SA, Goodlett DR, Nelson SD. Pro-CrossLink. Software Tool for Protein Cross-Linking and Mass Spectrometry. Anal Chem 2006;78(7):2145-9.

9. Higuchi T, Mechanism of sustained-action medication. Theoretical analysis of rates of release of solid drug dispersed in solid matrices. J. Pharm. Sci. 52 12 1963: 1145-1149.

10. Horvath A.L, Handbook of aqueous electrolyte Solutions, John Wiley and Sons, New York, 1985.

11. Huang SF, Chen JL, Yeh MK, Chiang CH. Physicochemical properties and in vivo assessment of timolol-loaded poly(D,L-lactide-co-glycolide) films for long-term intraocular pressure lowering effects. Ocular Pharmacol Ther, 2005 Dec;21(6):445-53.

12. Izutsu K, Aoyagi N.Effect of inorganic salts on crystallization of poly(ethylene glycol) in frozen solutions. Int J Pharm. 2005;288(1):101-8.

13. Jones DS, Andrew GP, Gorman SP. Characterization of crosslinking effects on the physicochemical and drug diffusional properties of cationic hydrogels designed as bioactive urological biomaterials. J Pharm Pharmacol. 2005;57(10):1251-59.

14. Melander W and Horvath C. Salt effect on hydrophobic interactions in precipitation and chromatography of proteins: an interpretation of the lyotropic series. Arch Biochem Biophys. 1977; 183(1):200-15.

15. Moelbert S, Normand B, De Los Rios P. Kosmotropes and chaotropes: modelling preferential exclusion, binding and aggregate stability Biophysical Chemistry 112: 45-57, 2004

16. Neagu A, Neagu M and Dér A. Fluctuations and the Hofmeister Effect. Biophysical Journal 2001 81:1285-1294

17. Nystrom B, Thuresson K and Lindmad B. Rheological and Dynamic Light-Scattering Studies on Aqueous Solutions of a Hydrophobically Modified Nonionic Cellulose Ether and Its Unmodified Analogue. Langmuir 1995;11:1994-2002

18. Peppas N.A and Sahlin JJ, A simple equation for the description of solute release. III. Coupling of diffusion and relaxation. Int J. Pharm. 57 (1989), pp. 169-172.

19. Pillay V and Fassihi R. Electrolyte-induced compositional heterogeneity: a novel approach for rate-controlled oral drug delivery. J Pharm Sci. 1999; 88(11):1140-8

20. Pillay V and Fassihi R. Probing the dynamics of matrix in the presence of electrolytes. Drg Deliv. 2001; 8(2):87-92.

21. Swenson J, Smalley MV, Hatharasinghe HLM, and Fragneto G. Langmuir Interlayer Structure of a Clay-Polymer-Salt-Water System. 2001, 17, 3813-3818.

22. Tanaka M and Takahashi K. Determination of the changes of the basic structures of silics species in dependence on the concentration of sodium chloriden by FAB-MS. Fresenius J Anal Chem. 2000; 368(8):786-90.

23. Young CR, Dietzsch C, Cerea M, Farrell T, Fegely KA, Rajabi-Siahboomi A, McGinity JW. Physicochemical characterization and mechanisms of release of theophylline from melt-extruded dosage forms based on a methacrylic acid copolymer. Int J Pharm. 2005;301(1-2):112-20.

24. Zhang Z, Chisti Y and Moo-Young M. Isolation of a recombinant intracellular beta-galactosidase by ammonium sulfate fractionation of cell homogenates. Bioseparation. 1995; 5(6):329-37.

## Claims

1. A monolithic drug delivery dosage form comprising a salted-out and crosslinked polymer having a pharmaceutically active agent disposed therewith **characterised in that** the polymer is poly-lactic co-glycolic acid and is crosslinked and salted-out with a crosslinking agent selected from the group consisting of: sodium chloride, aluminium chloride and calcium chloride, so as to entangle the pharmaceutically active agent with the poly-lactic co-glycolic acid.

2. The monolithic drug delivery dosage form according to claim 1, **characterised in that** the pharmaceutically active agent is melatonin.

3. A method of producing a monolithic drug delivery dosage form comprising a pharmaceutically active agent **characterised in that** the method includes the steps of salting-out and crosslinking poly-lactic co-glycolic acid with a crosslinking agent selected from the group consisting of: sodium chloride, aluminium chloride and calcium chloride.

4. The method according to claim 3, **characterised in that** said pharmaceutical active agent is melatonin.

## Patentansprüche

1. Monolithische Arzneimittel-Freisetzungs-Darreichungsform, die ein ausgesalztes und vernetztes Polymer umfasst, das ein pharmazeutisch aktives Mittel darauf abgelagert hat, darin charakterisiert, dass das Polymer poly-Milch-co-Glykol Säure ist und vernetzt und ausgesalzt ist mit einem Vernetzungsmittel ausgewählt aus der Gruppe bestehend aus: Natriumchlorid, Aluminiumchlorid und Kalziumchlorid, so dass sich das pharmazeutisch aktive Mittel mit der poly-Milch-co-Glykol Säure umschlingt.

2. Monolithische Arzneimittel-Darreichungsform nach Anspruch 1 darin charakterisiert, dass das pharmazeutisch aktive Mittel Melatonin ist.

**3.** Verfahren zur Herstellung einer monolithischen Arzneimittel-Darreichungsform, das ein pharmazeutisch aktives Mittel umfasst, darin charakterisiert, dass das Verfahren die Schritte Aussalzen und Vernetzung der poly-Milch-co-Glykol Säure mit einem Vernetzungsmittel ausgewählt aus der Gruppe bestehend aus: Natriumchlorid, Aluminiumchlorid und Kalziumchlorid enthält.

**4.** Verfahren nach Anspruch 3 darin charakterisiert, dass das pharmazeutisch aktive Mittel Melatonin ist.

**Revendications**

**1.** Forme galénique monolithique d'administration de médicament comprenant un polymère relargué et réticulé ayant un agent pharmaceutiquement actif se trouvant avec celui-ci, **caractérisée en ce que** le polymère est un acide poly(lactique-co-glycolique) et est réticulé et relargué avec un agent de réticulation choisi dans le groupe constitué par le chlorure de sodium, le chlorure d'aluminium et le chlorure de calcium, de sorte à entremêler l'agent pharmaceutiquement actif avec l'acide poly(lactique-co-glycolique).

**2.** Forme galénique monolithique d'administration de médicament selon la revendication 1, **caractérisée en ce que** l'agent pharmaceutiquement actif est la mélatonine.

**3.** Procédé de production d'une forme galénique monolithique d'administration de médicament comprenant un agent pharmaceutiquement actif **caractérisé en ce que** le procédé comprend les étapes de relargage et de réticulation d'un acide poly(lactique-co-glycolique) avec un agent de réticulation choisi dans le groupe constitué par le chlorure de sodium, le chlorure d'aluminium et le chlorure de calcium.

**4.** Procédé selon la revendication 3, **caractérisé en ce que** ledit agent pharmaceutiquement actif est la mélatonine.

**Figure 1:** Typical Force-Distance and Force-Time profiles of PLGA scaffolds for determining (a) energy absorbed (b) deformability modulus and (c) matrix resilience (N=10).

**Figure 2**: Selected scanning electron micrographs of PLGA scaffolds demonstrating the surface morphology of PLGA: salted-out with NaCl [(a) and (b)], $CaCl_2$, [(c) and( d)], and $AlCl_3$ [(e) and (f)]

**Figure 3:** Profiles depicting differences in the physicomechanical properties of PLGA scaffolds. Plot (a) Resilience (R), (b) Energy absorbed (E) and (c) Deformability modulus (DM).

**(a)**

**(b)**

**(c)**

**Figure 4:** Typical surface response plots depicting the effects of the independent formulation variables on the physicomechanical properties of the salted-out PLGA scaffolds, namely (a) Resilience (R) (%); (b) Energy absorbed (E) (J); and (c) Deformability modulus (DM) (N/mm). $AC= AlCl_3$, $CC= CaCl_2$, $SC= NaCl$. $-1=0\%^{w}/_v$ $0=5\%^{w}/_v$ $1=10\%^{w}/_v$.

**Figure 5:** Typical profiles used to construe the main and interactions effects of NaCl (a) and (d); CaCl₂ (b) and (e); AlCl₃ (c) and (f) for resilience in the presence of a combination of parameters.

**Figure 6:** Profiles demonstrating the correlation between experimental and fitted response values. R=Resilience; E=Energy absorbed; DM= Deformability modulus.

**Figure 7:** Five superimposed FTIR profiles depicting transitions from native PLGA to salted-out PLGA scaffolds. (a) = Native PLGA, (b)-(e) = PLGA salted-out with NaCl, $CaCl_2$, $AlCl_3$ and a combination of NaCl + $CaCl_2$ + $AlCl_3$ respectively.

**Figure 8:** Differential Scanning Calorimetry (DSC) profiles of native and salted-out PLGA scaffolds demonstrating thermal transitions of (a) native PLGA, (b) PLGA salted-out with NaCl, (c) $CaCl_2$, (d) $AlCl_3$, and (e) a combination of NaCl, $CaCl_2$, and $AlCl_3$.

**Figure 9:** Release profiles of melatonin from polymer when drug was either (a) (b) non-crosslinked or (c) crosslinked during the formation of PLGA scaffolds.

**EP 2 124 902 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WEBBER, W. L. et al.** Characterization of soluble , salt loaded, degradable PLGA films and their release of tetracycline. *JOURNAL OF BIOMEDICAL MATERIALS RESEARCH PART A,* 06 December 1998, vol. 41 (1
- **WEBBER, W. L. et al.** Characterization of soluble , salt loaded, degradable PLGA films and their release of tetracycline. *JOURNAL OF BIOMEDICAL MATERIALS RESEARCH PART A,* 06 December 1998, vol. 41 (1), 18-29
- **AVGOUSTAKIS K.** Pegylated poly(lactide) and poly(lactide-co-glycolide) nanoparticles: preparation, properties and possible applications in drug delivery. *Curr Drug Delivery,* 2004, vol. 1 (4), 321-33
- **ARAKAWA T ; TIMASHEFF SN.** Mechanism of poly(ethylene glycol) interaction with proteins. *Biochemistry,* 1985, vol. 24, 6756-6762
- **ASTHAGIRI D ; PRATT LR ; PAULAITIS ME ; REMPE SB.** Hydration Structure and Free Energy of Biomolecularly Specific Aqueous Dications, Including Zn2+ and First Transition Row Metals. *J. Am. Chem. Soc.,* 2004, vol. 126 (4), 1285-1289
- **BOLEN DW ; BASKAKOV IV.** The osmophobic effect: natural selection of a thermodynamic force in protein folding. *J. Mol. Biol.,* 2001, vol. 310, 955-963
- **DAYAL P ; PILLAY V ; BABU RJ ; SINGH M.** Box-Behnken experimental design in the development of a nasal drug delivery system of model drug hydroxyurea: characterization of viscosity, in vitro drug release, droplet size, and dynamic surface tension. *AAPS PharmSciTech.,* 2005, vol. 6 (4), E573-85
- **DASHEVSKY A ; WAGNERK ; KOLTER K ; BODMEIER R.** Physicochemical and release properties of pellets coated with Kollicoat SR 30 D, a new aqueous polyvinyl acetate dispersion for extended release. *Int J Pharm.,* 2005, vol. 290 (1-2), 15-23
- **GALINSKI EA ; STEIN M ; AMENDT B ; KINDER M.** The kosmotropic (structure-forming) effect of compensatory solutes. *Comp. Biochem. Physiol.,* 1997, vol. 117A, 357-365
- **GAO Q ; XUE S ; DONEANU CE ; SHAFFER SA ; GOODLETT DR ; NELSON SD.** Pro-CrossLink. Software Tool for Protein Cross-Linking and Mass Spectrometry. *Anal Chem,* 2006, vol. 78 (7), 2145-9
- **HIGUCHI T.** Mechanism of sustained-action medication. Theoretical analysis of rates of release of solid drug dispersed in solid matrices. *J. Pharm. Sci,* 1963, vol. 52 (12), 1145-1149
- **HORVATH A.L.** Handbook of aqueous electrolyte Solutions. John Wiley and Sons, 1985
- **HUANG SF ; CHEN JL ; YEH MK ; CHIANG CH.** Physicochemical properties and in vivo assessment of timolol-loaded poly(D,L-lactide-co-glycolide) films for long-term intraocular pressure lowering effects. *Ocular Pharmacol Ther,* December 2005, vol. 21 (6), 445-53
- **IZUTSU K ; AOYAGI N.** Effect of inorganic salts on crystallization of poly(ethylene glycol) in frozen solutions. *Int J Pharm.,* 2005, vol. 288 (1), 101-8
- **JONES DS ; ANDREW GP ; GORMAN SP.** Characterization of crosslinking effects on the physicochemical and drug diffusional properties of cationic hydrogels designed as bioactive urological biomaterials. *J Pharm Pharmacol.,* 2005, vol. 57 (10), 1251-59
- **MELANDER W ; HORVATH C.** Salt effect on hydrophobic interactions in precipitation and chromatography of proteins: an interpretation of the lyotropic series. *Arch Biochem Biophys.,* 1977, vol. 183 (1), 200-15
- **MOELBERT S ; NORMAND B ; DE LOS RIOS P.** Kosmotropes and chaotropes: modelling preferential exclusion, binding and aggregate stability. *Biophysical Chemistry,* 2004, vol. 112, 45-57
- **NEAGU A ; NEAGU M ; DÉR A.** Fluctuations and the Hofmeister Effect. *Biophysical Journal,* 2001, vol. 81, 1285-1294
- **NYSTROM B ; THURESSON K ; LINDMAD B.** Rheological and Dynamic Light-Scattering Studies onAqueous Solutions of a Hydrophobically Modified Nonionic Cellulose Ether and Its Unmodified Analogue. *Langmuir,* 1995, vol. 11, 1994-2002
- **PEPPAS N.A ; SAHLIN JJ.** A simple equation for the description of solute release. III. Coupling of diffusion and relaxation. *Int J. Pharm,* 1989, vol. 57, 169-172
- **PILLAY V ; FASSIHI R.** Electrolyte-induced compositional heterogeneity: a novel approach for rate-controlled oral drug delivery. *J Pharm Sci.,* 1999, vol. 88 (11), 1140-8
- **PILLAY V ; FASSIHI R.** Probing the dynamics of matrix in the presence of electrolytes. *Drg Deliv.,* 2001, vol. 8 (2), 87-92

- **SWENSON J ; SMALLEY MV ; HATHARASINGHE HLM ; FRAGNETO G.** *Langmuir Interlayer Structure of a Clay-Polymer-Salt-Water System.,* 2001, vol. 17, 3813-3818
- **TANAKA M ; TAKAHASHI K.** Determination of the changes of the basic structures of silics species in dependence on the concentration of sodium chloridenby FAB-MS. *Fresenius J Anal Chem.,* 2000, vol. 368 (8), 786-90
- **YOUNG CR ; DIETZSCH C ; CEREA M ; FARRELL T ; FEGELY KA ; RAJABI-SIAHBOOMI A ; MCGINITY JW.** Physicochemical characterization and mechanisms of release of theophylline from melt-extruded dosage forms based on a methacrylic acid copolymer. *Int J Pharm.,* 2005, vol. 301 (1-2), 112-20
- **ZHANG Z ; CHISTI Y ; MOO-YOUNG M.** Isolation of a recombinant intracellular beta-galactosidase by ammonium sulfate fractionation of cell homogenates. *Bioseparation,* 1995, vol. 5 (6), 329-37